# EUROPÄISCHE PATENTSCHRIFT

(11) **EP 1 883 387 B1**
(45) Veröffentlichungstag und Bekanntmachung des Hinweises auf die Patenterteilung: **05.10.2011**
(21) Anmeldenummer: 06755249.7
(22) Anmeldetag: 18.05.2006
(51) Int. Cl.: A61J 1/06, B65D 1/09

(54) **Behälter für zu verabreichende flüssige Medikamente**
Container for liquid medicaments to be administered
Contenant pour médicaments liquides pour être administrés

(30) Priorität: 24.05.2005 EP 05104386
(43) Veröffentlichungstag der Anmeldung: 06.02.2008
(73) Patentinhaber: VIFOR (INTERNATIONAL) AG, 9001 St. Gallen (CH)
(72) Erfinder: WEIBEL-FURER, Ludwig, CH-9104 Waldstatt (CH); WEIBEL-FURER, Dominique, CH-9104 Waldstatt (CH)
(74) Vertreter: Gille Hrabal Struck Neidlein Prop Roos
(86) Internationale Anmeldenummer: PCT/EP2006/062415
(87) Internationale Veröffentlichungsnummer: WO 2006/125747

(56) Entgegenhaltungen:
- DE-A1- 19 518 426
- US-A- 3 114 369
- US-A- 3 989 045
- US-A- 4 328 912
- US-A1- 2003 010 795
- US-A1- 2003 015 605
- US-B1- 6 379 342
- US-B1- 6 585 693

## Beschreibung

Die vorliegende Erfindung betrifft einen Behälter für die Aufnahme flüssiger Medikamente und deren Abgabe in eine Verabreichungsvorrichtung und die Verwendung sowie die zugehörige Verabreichungsvorrichtung. Bei den Medikamenten handelt es sich beispielsweise um parenteral zu verabreichende Medikamente, aber auch orale oder topische Medikamente.

Der erfindungsgemäße Behälter weist eine besonders vorteilhafte Ausgestaltung der Umhüllung auf.

Bei der parenteralen, d.h. den Darm eines Säugers umgehenden Verabreichung von Medikamenten geschieht dies meist durch Injektion oder Infusion. Unter einer Injektion versteht man die Verabreichung eines flüssigen, sterilen Medikamentes mittels Spritze und Hohlnadel direkt in das Gewebe oder Gefäßsystem unter Umgehung des Magen-Darm-Traktes. Bei der Infusion handelt es sich um langsames, meist tropfenweises Einfließen größerer (arzneimittelhaltiger) Flüsslgkeitsmengen in den Körper.

Medikamente, beispielsweise parenteral zu verabreichende Medikamente, werden im Allgemeinen nach ihrer Zubereitung in einen Behälter abgefüllt, der eine oder mehrere Portionen/Dosen aufnehmen kann. Dieser Behälter wird als Primärpackung bezeichnet.

Als Primärpackung sind Glas-Ampullen bekannt die mit einer speziellen Ampullensäge zu öffnen sind oder die zum Zwecke der Öffnung mit einer Sollbruchstelle versehen sind. Der Inhalt aus der Ampulle muss in einen für die Verabreichung geeigneten Behälter, wie z.B. eine Spritze, transferiert werden. Ferner muss meist noch Restflüssigkeit in der Ampulle verbleiben, da es ansonsten bei dem Aufziehen der Spritze nachteilig zum Ansaugen von Luft kommen kann. Darüber hinaus ist ein späterer Verschluss der Ampulle mit der darin enthaltenden Restflüssigkeit praktisch unmöglich.

Als weiterer Behälter ist aus dem Stand der Technik eine sogenannte Stechampulle (Vial) bekannt. Diese wird mit einer Spritze an einer dafür vorgesehenen Stelle eingestochen, und die Spritze wird dann mit der im Behälter befindlichen Flüssigkeit aufgezogen. Dieses mit diesem Behälter verbundene Verfahren ist sehr aufwendig, da die Ampulle nicht unmittelbar zur Verabreichung verwendet werden kann, sondern der Inhalt aus der Ampulle in einen für die Verabreichung geeigneten Behälter, wie die zuvor genannte Spritze, transferiert werden muss. Das Öffnen oder Anstechen von Ampullen und ein anschließendes notwendiges Transferieren in einen für die Verabreichung geeigneten Behälter erweist sich z.B. in Notsituationen bei denen die Verabreichung unter Zeitdruck zu erfolgen hat, als nachteilig.

Heute wird auch unmittelbar in eine Spritze abgefüllt, die zur Verabreichung mit einer aufgesteckten oder eingeklebten Kanüle versehen ist. Durch Pressen des im Spritzenbehälter beweglich gelagerten Kolbens wird die Flüssigkeit aus dem Behälter durch die Kanüle in den Verabreichungsort injiziert. Die Spritze weist den Nachteil auf, dass sie aufgrund des mehrteiligen Aufbaus vergleichsweise teuer ist. Zur Aufrechterhaltung der Funktionsfähigkeit auch nach längerer Lagerdauer sind Kolben und Spritzenbehälter mit Beschichtungen, bspw. mit Silikon, versehen. Damit wird eine Substanz zusammen mit dem Medikament gelagert und verabreicht, die mit der eigentlichen Wirkung des Medikaments nichts zu tun hat und sich z.B. bei Medikamenten mit einem hohen pH Wert sogar nachteilig auswirken kann.
Nebst den parenteralen Medikamenten, welche i.a. in Ampullen, Vials oder Fertigspritzen, sowie in Infusionsflaschen bzw. -beuteln abgefüllt werden, werden auch teilweise nicht parenteral verabreichte Medikamente in Ampullen oder Vials aufbewahrt, da sie vor der Verabreichung z.B. mit Wasser gemischt werden müssen.

Aus der US 4 926 915 A ist ein Behälter in Form einer Tube bekannt. Die darin offenbarte Tube stellte eine Vereinfachung der Ampulle oder Stechampulle dar, da eine Spritze direkt angesetzt und aufgezogen werden kann. Als Verbindungsmittel zu einer Tube wird eine konisch nach innen spitz zulaufende Fläche vorgeschlagen.

Die GB 800 455 A offenbart die Verbindung zweier Tuben zu einem gegenseitigen Inhaltsausstoß. Die Verbindung mit einer Verabreichungsvorrichtung ist nicht vorgesehen.

Die US 4 926 915 A offenbart u.a. einen tubenförmigen Behälter mit einem Luer-Anschluss zur Verbindung mit einer Ampulle, Die Verbindung mit einer Verabreichungsvorrichtung ist ebenfalls nicht vorgesehen.

In US 2003/0010795 A1 ist ein Behälter gezeigt, der so ausgestaltet ist, dass eine ausgelöste Abgabe einer Flüssigkeit selbstständig fortgesetzt wird.

Die US 4 328 912 A beschreibt einen kollabierbaren Behälter, der in der Lage ist, die Abgabe einer Flüssigkeit selbstständig aufgrund einer spezifischen Gestaltung eines Ventils durchzuführen,

Die US 2003/0015605 A1 beschreibt einen mit einer Flüssigkeit gefüllten Behälter, bei dem unter Druckeinwirkung erst nach Überwindung eines Anfangswiderstands ein Austritt schlagartig ausgelöst wird.

In den Druckschriften US 6,585,693 B1 sowie DE 195 18 426 A1 wird ein Behälter für die Aufnahme und Abgabe eines Im Behälter befindlichen Medikaments beschrieben, wobei der Behälter eine bis auf eine Öffnung zur Abgabe des Medikaments verschlossene, tubenförmige Umhüllung aufweist, Der Behälter ist so gestaltet, dass unter Änderung von wenigstens einem Bereich der Umhüllung eine Abgabe des Medikaments erfolgt. Der Behälter weist eine Hohlnadel im Bereich der Öffnung auf, und die Hohlnadel ist einstückig mit der tubenförmigen Umhüllung ausgebildet.

Vor dem Hintergrund der oben beschriebenen Nachteile ist es daher Aufgabe der vorliegenden Erfindung, einen Behälter für die Aufnahme und Abgabe flüssige Medikamente bzw. einen Behälter mit zugehöriger Verabreichungsvorrichtung bereitzustellen, welcher jeweils die Verabreichung eines Medikaments sowie die Verbindung mit einer Verabreichungsvorrichtung erleichtert und vergleichsweise einfach aufgebaut ist.

Diese Aufgabe wird durch den Behälter des Anspruchs 1 gelöst. Vorteilhafte Ausgestaltungen ergeben sich aus den Unteransprüchen. Eine vorteilhafte Verwendung ist Gegenstand des nebengeordneten Anspruches.

Der erfindungsgemäße Behälter zur Aufnahme und Abgabe eines Medikaments ist mit einer bis auf eine Öffnung zur Abgabe des Medikaments verschlossenen und einstückigen Umhüllung versehen. Der Behälter ist ferner derart ausgestaltet, dass unter Änderung der ganzen oder von einem Bereich der Umhüllung eine Abgabe des Medikaments aus der Öffnung erfolgt. Das Medikament liegt beispielsweise in einer Lösung oder sonstigen Flüssigkeit vor, wobei die Viskosität unterschiedlich gewählt sein kann. Beispielsweise handelt es sich um ein parenteral, oral oder topisch zu verabeichendes Medikament.

Erfindungsgemäß ist die Änderung der Umhüllung mit der Abgabe des Medikaments verbunden. Beispielsweise ist die Umhüllung aufgrund der Materialwahl und insbesondere bei einer vergleichsweise großvolumigen Umhüllung kollabierend ausgestaltet, das heißt, dass bei der Abgabe des Medikaments mittels Kapiflarkräfte und / oder aufgrund der Schwerkraftwirkung aufgrund des sich im Behälter ausbildenden Unterdrucks es zur wenigstens teilweisen Kollabierung der Umhüllung und somit Änderung der Umhüllung kommt.

Einstückigkeit ist so zu verstehen dass die Umhüllung nicht mehrteilig aufgebaut ist, Die Umhüllung besteht beispielsweise aus einem Material mehreren Materiallen und oder weist in verschiedenen Bereichen unterschiedliche Zusammensetzungen auf. Die Einstückigkeit der Umhüllung sorgt gegenüber einem mehrteiligen Aufbau, wie bei einer Kolbenvorrichtung, für eine preiswerte Herstellung der Vorrichtung. Ferner erfolgt die Verabreichung besonders einfach, steril, schnell und störunanfällig, Aufgrund der Einstückigkeit ist beispielsweise gegenüber einer Konstruktion mit beweglichem Kolben die Anzahl der produktberührenden Elemente bzw, Materialien reduziert, so dass die Kompatibilität mit dem Medikament vorteilhaft und vergleichsweise einfach sichergestellt werden kann.

Der erfindungsgemäße Behälter umfasst ferner eine Hohlnadel im Bereich der Öffnung. In einer Ausführungsform dient die Hohlnadel als Verbindungsmittel zu einer Verabreichungsvorrichtung oder zu einem Behälter für eine medizinische Flüssigkeit. Durch die Hohlnadel kann die Verbindung schnell und sicher erfolgen. Bei den Verabreichungsvorrichtungen, zu denen mittels der Nadel eine flüssigkeitsleitende Verbindung herzustellen ist, handelt es sich um Mittel zur Infusion oder Injektion, die beispielsweise eine Membran oder Folie aufweisen, die mit der Hohlnadel zum Entleeren des Behälters in die Verabreichungsvorrichtung durchstoßen wird.

Es handelt sich in einer vorteilhaften Ausgestaltung der erfindungsgemäßen Vorrichtung um eine Verabreichungsvorrichtung, also um eine Infusions- oder Injektionsnadel oder -Kanüle und dient dazu, das Medikament über die Öffnung an einen Patienten zu verabreichen. Wird der zum Transport und der Lagerung verwendete Behälter gleichzeitig zur Verabreichung des Medikaments verwendet, entfällt vorteilhaft ein Transferiervorgang von einem Transportbehälter in einen für die Verabreichung bestimmten Behälter.

Die Hohlnadel dient in einer Ausgestaltung der direkten Verabreichung beispielsweise durch Injektion. Die Injektion erfolgt je nach Verabreichungsort intracutan, subcutan, intramuskulär, intravenös, intraarteriell, intrakardial, intraartikular, intrathekal oder intralumbal.

Die Umhüllung ist einstückig mit der Hohlnadel ausgebildet. Dadurch kann der Behälter besonders preiswert hergestellt werden. Dadurch kommt vorteilhaft bei der Abgabe des Medikaments kein weiteres Material mit dem Medikament in Berührung. Der Kontakt des Medikaments beschränkt sich während der Aufbewahrung und Abgabe auf ein Material bzw. eine Materialzusammensetzung. So kann besonders einfach sichergestellt werden, dass der Kontakt mit Materialien des Behälters sich nicht nachteilig, beispielsweise pH-Wert ändernd, auf das im Behälter enthaltende Medikament auswirkt. Ferner kann so der Behälter besonders einfach, kostengünstig und schnell hergestellt werden, was in herstellungstechnischer Hinsicht von Vorteil ist.

Der erfindungsgemäße Behälter ist so ausgestaltet, dass die Abgabe des Medikaments durch Druck auf wenigstens einen Außenbereich der Umhüllung erreicht oder wenigstens ausgelöst wird. Beispielsweise ist die Umhüllung derart gestaltet, dass durch einen Daumendruck auf den zugehörigen Außenbereich der Umhüllung eine Abgabe des Medikaments bewirkt wird. Dadurch wird eine besonders reproduzierbare, dosierte Abgabe des Medikaments, das beispielsweise in flüssiger Form mit unterschiedlichster Viskosität vorliegt, ermöglicht. Beispielsweise wird der für eine Injektion erforderliche Injektionsdruck, durch den auf die Umhüllung einwirkenden Druck generiert, zusätzliche Mittel zur Druckerzeugung, beispielsweise eine Pumpe, können somit vorteilhaft entfallen. Beispielsweise ist der Behälter röhrenförmig ausgebildet. Ein Druck auf die röhrenförmige Umhüllung löst eine Volumenverkleinerung aus. Die zugehörige Volumenverkleinerung bewirkt eine Verdrängung des sich im Behälter aufgenommenen Medikaments aus dem Behälter und damit ein Austreten und damit eine Abgabe über die Öffnung. Je nach gewählter Härte der Umhüllung kann die Charakteristik des aufzubringenden Druckes vorteilhaft variiert werden.

Der Behälter ist in einer weiteren vorteilhaften Ausführungsform so ausgestaltet, dass nach der Auslösung der Abgabe durch den Druck die Abgabe des Medikaments selbständig fortgesetzt wird. Beispielsweise wird dies durch eine Ausgestaltung erreicht, bei der ein relativ harter und elastischer Umhüllungsabschnitt bei Erreichen einer gewissen Außenkrümmung nach dem Knackfrosch-Prinzip federartig in die entgegengesetzte, in das Behälterinnere weisende Krümmung überspringt und so aufgrund der Federcharakteristik des Umhüllungsabschnitts ein genau definiertes Medikamentenvolumen abgibt. Dadurch wird einerseits eine selbständige und andererseits wohl dosierte Abgabe erreicht.

Der Behälter weist eine tubenförmige Umhüllung auf, "Tubenförmig" im Sinne der Erfindung ist so zu verstehen, dass der Behälter im Wesentlichen infolge der Verschweißung zu seinem der Öffnung gegenüberliegendem Ende spitz zuläuft. Durch diese Art der Ausgestaltung wird eine besonders leichte Entleerung des Behälters und damit Abgabe des Medikaments erreicht. Eine Tube wird beispielsweise durch eine Röhre erzeugt, bei der ein Ende durch flaches Verschweißen oder Falzen jeweils in Abhängigkeit des verwendeten Materials verschlossen ist. Der vor dem Verschweißen oder Falzen bestehende Zugang ins Innere des tubenförmigen Behälters dient in einer Ausgestaltung vorteilhaft zudem als Zuführmöglichkeit für die Befüllung mit dem Medikament, das nachfolgend im Behälter aufgenommen wird und über die erfindungsgemäß vorgesehene Öffnung gegebenenfalls abgegeben wird.

Bei einer weiteren vorteilhaften Ausführungsform umfassen Mittel zur Verbindung der Öffnung mit der Verabreichungsvorrichtung ein Arretiermittel gegen ein unbeabsichtigtes Lösen der Verbindung auf. Beispielsweise ist eine Schraubvorrichtung mit einer Einrastfunktion versehen, die die Schreubverbindung im eingeschraubten Zustand durch ein einrastendes Federelement zusätzlich sichert. Ein Lösen ist erst nach Überwinden dieser, mit der Einrastung verbundenen, Federwirkung möglich und verhindert so weitgehend ein unbeabsichtigtes Lösen der Verbindung.

Gemäß einer weiteren vorteilhaften Ausführungsform des erfindungsgemäßen Behälters weisen die Mittel zur lösbaren Verbindung zwei konisch geformte zueinander komplementäre Flächen auf, Dadurch wird eine einfache und gleichzeitig ausreichend dichte Verbindung erreicht. Bevorzugt ist der Behälter mit einem Fortsatz mit konisch zur Öffnung spitz zulaufender Außenfläche versehen.

Gemäß einer Ausführungsform handelt es sich um einen Luer-Anschluss (Luer cone oder Luer lock). Ein so genannter Luer-Anschluss ist in der ISO 594/1 definiert. Im Allgemeinen handelt es sich, um einen als "male" bezeichneten Anschluss. Durch Vorsehen eines solchen Anschlusses am Behälter werden bei der Verabreichung des Medikaments mehrere Arbeitsschritte eingespart werden.

Die Verbindungsmittel sind gemäß einer weiteren vorteilhaften Ausgestaltung wenigstens teilweise einstückig mit dem Behälter ausgebildet. Dadurch kann der Behälter besonders einfach, kostengünstig und schnell hergestellt werden, was in herstellungstechnischer Hinsicht von Vorteil ist.

Die Einstückigkeit wird beispielsweise in einem einzigen Herstellungsschritt oder wie folgt erreicht: in einem ersten Schritt der Herstellung werden zwei Stücke hergestellt, weiche anschließend verschweißt oder zusammengesteckt werden. Der Abfüller wird dann mit einem einstückigen Teil beliefert, um dies mit dem Medikament zu befüllen. Eine Zusammenführung zweier Teile ist von Vorteil, wenn auf vorhandene Spritzformen zurückgegriffen wird, bei einem Herstellverfahren, bei dem Kunststoff- oder Laminatschläuche, die den eigentlichen Behälter bilden, an ein Kopfteil mit den Verbindungsmitteln angeschweißt werden oder wenn die Verbindungsmittel eine besondere Komplexität aufweisen.

Der Behälten weist ein Volumen von weniger als 5ml. Ein Volumen von weniger als 5ml erweist sich als besonders vorteilhaft, da ein Behälter, insbesondere eine Tube ,durch einfachen Druck mit dem Daumen in einem Zug effizient und gleichmäßig entleerbar ist. Es hat sich gezeigt, dass dadurch die Handhabung des Behälters und damit die Medikamentverabreichung besonders einfach und effizient ist.

Der Behälter besteht in einer weiteren vorteilhaften Ausführungsform aus Kunststoff, insbesondere Polypropylen, PE, wie LDPE oder PET. Weiter verwendbar sind PVC, PVF etc. Heute werden auch neue, verschweißbare Kunststoffe entwickelt, welche für Medikamente vorteilhaft einsetzbar sind. Die erfindungsgemäße Kunststoffauswahl ist nur insoweit eingeschränkt, dass dadurch die Herstellbarkelt des erfindungsgemäßen Behälters gewährleistet ist. Dadurch kann der Behälter preiswert hergestellt werden. Ferner kann aufgrund der vergleichsweise niedrigen Schweiß- und Schmelztemperatur das Verschließen des Behälters nach erfolgter Abfüllung besonders medikamentschonend erfolgen. Darüber hinaus kann so leicht die Chargen- und / oder Verfallsinformation leicht auf die Außenfläche des Behälters eingeprägt werden. Diese Beschriftungen sind so von hoher Dauer.

Der Behälter besteht bei einer weiteren vorteilhaften Ausführungsform aus einem Laminat. Dadurch bestehen weitere Möglichkeiten der Kompatibilität, Beispielsweise kann in den Behältermantel eine Aluminiumfolie eingelegt werden, weiche selbst nicht produktberührend ist, jedoch eine erhöhte Sauerstoffbarriere darstellt und so die Produktstabilität günstig beeinflusst. Weiter können vorgedruckte Etiketten, welche ihrerseits wiederum Barrierefunktion aufweisen können, in den Mantel eingelegt werden (in mold Labelling). Dadurch entfällt sogar das Aufkleben von Etiketten, und es wird kein Kleber notwendig, welcher durch Diffusion ins Produkt gelangen könnte. Gemäß einer weiteren vorteilhaften Ausführungsform ist der Behälter wenigstens teilweise transparent ausgebildet. Dadurch kann vorteilhaft der Füllstand bzw, die Entleerung kontrolliert werden.

Der Behälter ist gemäß einer weiteren vorteilhaften Ausgestaltung beschichtet. Beispielsweise ist die mit dem Medikament in Berührung stehende innere Fläche der Umhüllung mit einer chemisch neutralen Beschichtung, wie Siliziumoxid beschichtet. Dadurch wird erreicht, dass der Behälter aufgrund der Beschichtung, in dieser Hinsicht glasähnliche Eigenschaft erreicht, ohne die mit Glas verbundenen Nachteile aufzuweisen: beispielsweise Verletzungsgefahr beim Öffnen und Entsorgen von Glasampullen usw..

Die Umhüllung des Behälters ist wenigstens teilweise elastisch ausgebildet.

Die Mittel zur Verbindung können ferner gemäß einer weiteren Ausführungsform mit einem dafür bestimmten Verschluss zusammenwirken, so dass ein besonders leichtes Verschließen des erfindungsgemäßen Behälters, beispielsweise im Gegensatz zu den eingangs beschriebenen Ampullen, erreicht wird. Der Verschluss ist beispielsweise ein separates Kunststoffspritzgussteil, das auf die zuvor beschriebene Hohlnadel aufgeschraubt oder aufgesteckt wird. In einer weiteren Ausführungsform ist der Verschluss einstückig mit der Umhüllung verbunden und wird zusammen mit dem Behälter in einem Verarbeitungsschritt hergestellt, um das im Behälter aufgenommene Medikament vor Kontamination zu schützen. Dadurch kann der Behälter samt Verschluss einfach und kostengünstig hergestellt werden, Die Verschlussmittel umfassen beispielsweise Schraub- oder Steckkappen und an die Umhüllung angeschweißte und mit Sollbruchstelle versehene Abschnitte.

Das Verfahren zur Herstellung des Behälters weist einen Schritt auf, bei dem in einem Kunststoffspritzguss-, Kunststoffspritzblas- oder einem Kunststoffextrusionsblasschritt der Behälter hergestellt wird. Dadurch kann der Behälter preiswert, gegebenenfalls zusammen mit dem Verschluss und / oder den Mitteln zur lösbaren Verbindung mit den Verabreichungsvorrichtungen, hergestellt werden. In einem weiteren Herstellverfahren werden Mantel und Kopfteil separat hergestellt und miteinander verschweißt. Ferner kann die für den Abfüller und Anwender vorteilhafte Einstückigkeit dadurch erreicht werden, dass zwei Teile vom Hersteller separat hergestellt, aber bereits von diesem zu einem Stück zusammengefügt (z.B. gesteckt und anschließend verschweißt) werden.

### Zu den Figuren:

Die Figuren 1a und 1b zeigen Ausführungsbeispiele des erfindungsgemäßen Behälters.

Die Figuren 2a und 2b zeigen eine Form des erfindungsgemäßen Behälters, wobei Bereiche der Umhüllung selbst kollabierend ausgeführt sind.

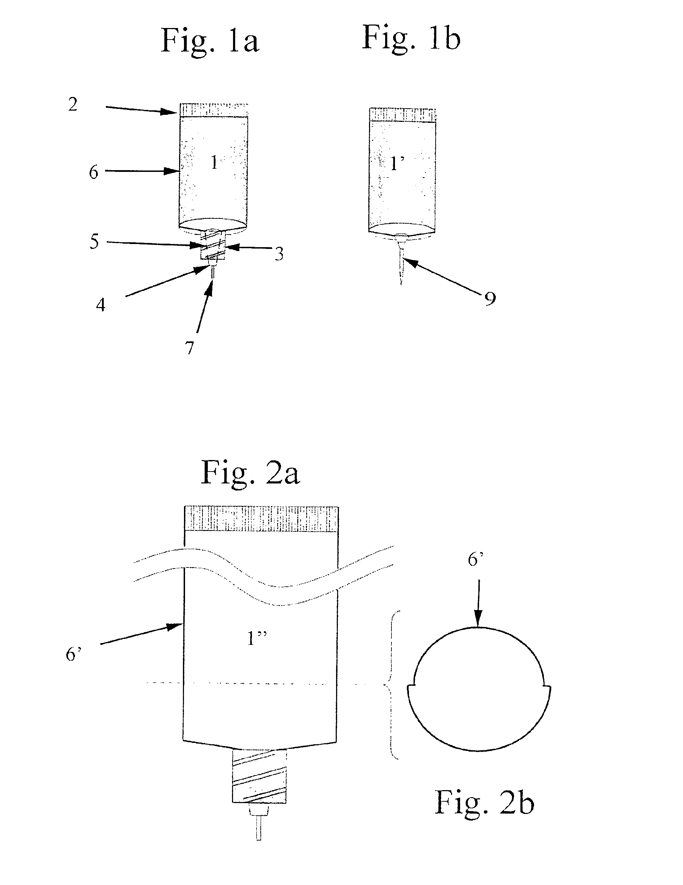

Figur 1a zeigt eine erste Ausführungsform des erfindungsgemäßen Behälters 1 mit einer tubenförmigen, einstückigen Umhüllung 6. In der Umhüllung 6 ist eine, ein (i.a. parenterales) Medikament beinhaltende, Flüssigkeit (nicht dargestellt) enthalten. Das (i.a. parenterale) Medikament bzw. die Flüssigkeit wird über die Öffnung 4 aus dem Behälter 1 abgegeben, indem im Bereich der 1 auf die Umhüllung 6 beispielsweise mit den Fingern gepresst wird. Die Umhüllung 6 ist auf der der Öffnung 4 gegenüber liegenden Seite mit einer eben verlaufenden Verschweißung 2 luft- und flüssigkeitsdicht verschlossen, wodurch sich die Tubenform ergibt. Die Verbindungsmittel der Öffnung 4 des Behälters mit einer nicht gezeigten Verabreichungsvorrichtung umfassen einen an der Umhüllung ausgebildeten konischen Fortsatz 5. Dessen Verlauf ist in der Fig. 1a gestrichelt angedeutet, da er teilweise von einer mit Innengewinde versehenen Hülse 3 umgeben ist, die ebenfalls Teil der Verbindungsmittel ist. Der konische Fortsatz kann ohne die Hülse vorgesehen sein und bildet somit einen sogenannten Luer male - Anschluss. Ist die Hülse mit Innengewinde vorgesehen, ergibt sich ein Aufbau, der einem sogenannten Luer lock Anschluss entspricht. Der konisch verlaufende Fortsatz 5 als Teil der Verbindungsmittel dient mit Verbindung eines dazu komplementären Verbindungselements einer Infusions- bzw. Injektionsnadel als Verabreichungsvorrlchtung (in der Abbildung nicht dargestellt), um diese einerseits flüssigkeitsleitend, andererseits dicht und lösbar zu verbinden.

Die Öffnung 4 ist beispielsweise an der Austrittsstelle mit einer Sollbruchstelle, wobei ein beliebig ausgeformtes Bruchhilfestück 7 angespritzt wird, oder mit einer direkt angegossenen Folie oder Membran verschlossen, welche z.B. mit einem spitzen Gegenstand durchstochen werden kann.

Fig. 1b zeigt eine weitere Ausführungsform 1' des erfindungsgemäßen, tubenförmigen Behälters. Dieser unterscheidet sich vor allem von der Ausführungsform 1 durch die andersartige Gestaltung der Umhüllung im Bereich der Öffnung. Ferner ist die Hohinadel 9 gezeigt, die zur Verbindung des Behälters mit einer Verabreichungsvorrichtung, also zur Einbringung desselben z.B. in einen Infusionsbeutel, dient. Die Hohinadel ist einstückig mit der Umhüllung ausgebildet.

Die Figuren 2a und 2b zeigen eine weitere Ausführungsform 1" des erfindungsgemäßen Behälters. Dieser ist mit einer Umhüllung 6' versehen, die bei der Abgabe des Medikaments das Knackfrosch-Prinzip ausnutzt. In Figur 2b ist die Umhüllung 6' im Schnitt dargestellt. Die in der Figur gezeigte obere Hälfte der Umhüllung springt nach innen elastisch über, nachdem nach anfänglichem äußeren Druck eine gewisse Krümmung überschritten ist. Diese nach innen gerichtete Federwirkung bewirkt einen Verdrängungseffekt auf das in der Umhüllung 6' befindliche Medikament und führt zu einer Abgabe des Medikaments über die Öffnung. Aufgrund der vorgegebenen Federwirkung wird reproduzierbar selbständig nach dem ersten, auslösenden Druck auf die Umhüllung 6' eine Abgabe bewirkt. Somit wird immer die gleiche Flüssigkeitsmenge ausgestoßen. Im Querschnitt ist ersichtlich, dass die obere Hälfte der Umhüllung nach Druck in die andere Hälfte eingepasst wird und so auch nicht mehr in die ursprüngliche Form zurückschnellen kann.

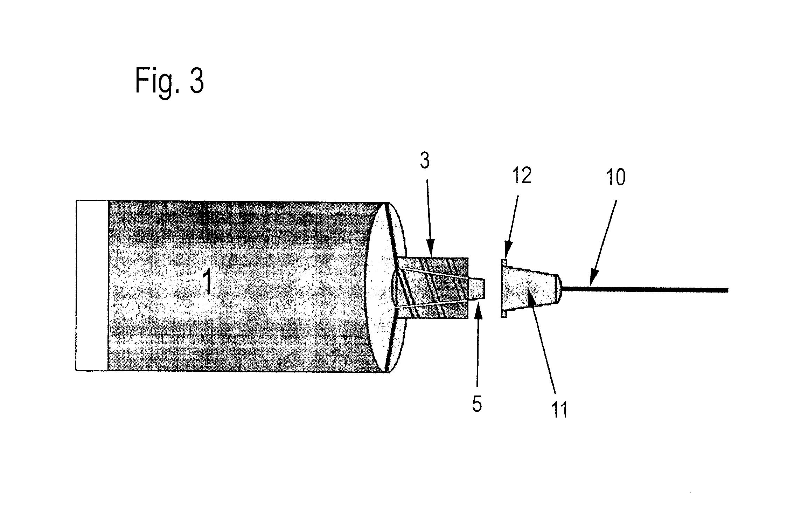

Figur 3 zeigt die Ausführungsform, die in Figur 1 gezeigt ist, mit zugehöriger Infusions- oder Injektionsnadel 10. Das an der Infusions- oder Injektionsnadel 10 angebrachte Verbindungsmittel 12 weist eine Innenfläche auf, die konisch nach innen derart spitz zuläuft, dass sie komplementär zur Außenfläche 5 des Verbindungsmittels des Behälters 1 ist. Durch Eingriff der beiden Flächen der Verbindungsmittel 5, 12 wird eine lösbare Verbindung zwischen dem Behälter 1 und der Nadel 10 hergestellt. Diese Verbindung wird durch eine Schraubverbindung zwischen dem Innengewinde der Hülse 3 und dem am Verbindungsteil 11 der Nadel vorgesehenen Außengewinde 12 zusätzlich fixiert.

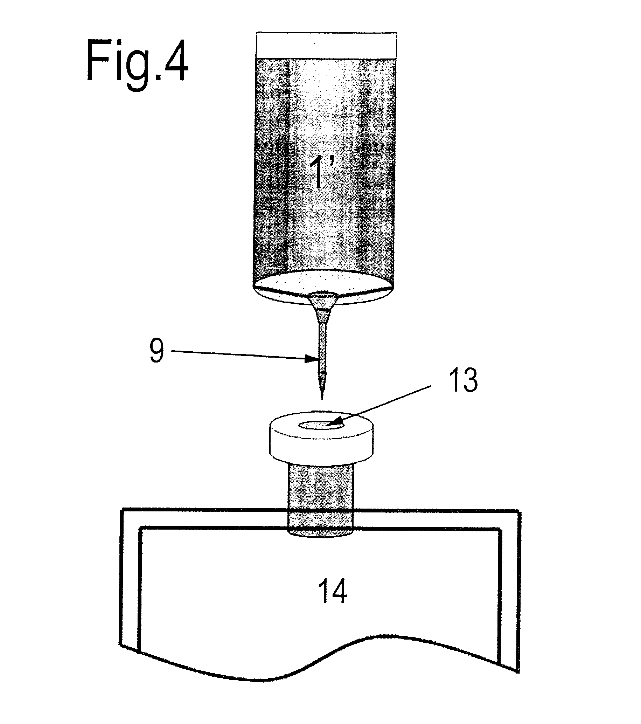

Figur 4 zeigt die Verwendung des Behälters 1' aus Fig. 1b. Die Hohlnadel 9 dient der Verbindung zu einem Infusionsbeutel oder einer Infusionsflasche 14, wobei eine Folie oder Membran 13 des Infusionsbeutels oder der Infusionsflasche 14 durchstoßen wird, um das im Behälter 1' befindliche Medikament in den Beutel oder die Flasche 14 einzubringen.

## Patentansprüche

1. Behälter (1, 1', 1") für zu verabreichende flüssige Medikamente, Insbesondere parenterale Medikamente, der eine bis auf eine Öffnung (7) zur Abgabe des Medikaments verschlossene, einstückige tubenförmige Umhüllung (6, 6') aufweist und so gestaltet ist, dass unter Änderung von wenigstens einem Bereich der Umhüllung (6, 6') eine Abgabe des Medikaments erfolgt, der Behälter ferner eine Hohlnadel (9, 10) im Bereich der Öffnung (7) aufweist und die Hohlnadel (9) einstückig mit der tubenförmigen Umhüllung (6, 6') ausgebildet ist,
**dadurch gekenntzeichnet**, dass der Behälter (1, 1', 1") ein Volumen von weniger als 5ml aufweist und die Umhüllung (6, 6') des Behälters (1, 1', 1") wenigstens teilweise elastisch ausgebildet ist sowie einen nach außen gekrümmten relativ harten und elastischen Abschnitt aufweist, der unter äußerem Druck nach Überschreiten seiner Krümmung in das innere der Umhüllung elastisch überspringt und auf das im Behälter befindliche Medikament dauerhaft einen Verdrängungseffekt ausübt, so dass der Behälter durch einfachen Druck mit dem Daumen in einem Zug entleerbar ist und so dass nach der Auslösung der Abgabe durch den Druck die Abgabe des Medikaments selbständig fortgesetzt wird.

2. Behälter gemäß Anspruch 1, **dadurch gekennzeichnet, dass** er infolge seiner Verschweißung ausgehend von seinem Ende mit der Öffnung (7) zum anderen verschweißten Ende (2) hin spitz zuläuft.

3. Behälter (1, 1', 1") gemäß Anspruch 1 oder 2, **dadurch gekennzeichnet, dass** er aus Kunststoff, Insbesondere Polypropylen, PE, wie LDPE oder PET, besteht.

4. Behälter (1, 1', 1") gemäß einem der Ansprüche 1 bis 3, **dadurch gekennzeichnet, dass** er ein Laminat aufweist.

5. Behälter (1, 1', 1") gemäß einem der Ansprüche 1 bis 4, **dadurch gekennzeichnet, dass** er wenigstens teilweise transparent ausgebildet ist.

6. Behälter (1, 1', 1") gemäß einem der Ansprüche 1 bis 5, **dadurch gekennzeichnet, dass** er teilweise chemisch neutral beschichtet ist.

7. Verfahren zum Entleeren des Behälters (1, 1', 1") gemäß einem der Ansprüche 1 bis 6, **dadurch gekennzeichnet, dass** der Behälter durch Druck mit dem Daumen auf den Behälter in einem Zug entleert wird.

## Claims

1. A container (1, 1', 1") for fluid drugs to be applied, especially parenteral drugs, comprising an integral tubular envelope (6, 6') which is sealed except for one opening (7) for delivery of the drug, and which is designed such that delivery of the drug will be accomplished by varying at least one region of the envelope (6, 6'), which container moreover comprises a hollow needle (9, 10) in the region of the opening (7), and which hollow needle (9) is formed integrally with the tubular envelope (6, 6'),
**characterised in that** the container (1, 1', 1") has a capacity of less than 5 ml and the envelope (6, 6') of the container (1, 1', 1 ") is at least partially formed in a flexible manner, as well as having a relatively hard and flexible section bent toward the outer side which under exterior pressure will flexibly pass into the interior of the envelope after exceeding its bend and will permanently apply a displacing action to the drug which is present in the container, such that the container can be emptied in one step by simple thumb pressing and after initiating delivery by exerting pressure delivery of the drug will proceed independently.

2. The container according to claim 1 **characterised in that** due to its welding it is tapered from its opening end (7) to the other welded end (2).

3. The container (1, 1', 1") according to one of the claims 1 or 2, **characterised in that** it is comprised of plastics, especially polypropylene, PE, such as LDPE or PET.

4. The container (1, 1', 1") according to one of the claims 1 to 3, **characterised in that** it has a laminate.

5. The container (1, 1', 1") according to one of the claims 1 to 4, **characterised in that** it is at least partially formed transparently.

6. The container (1, 1', 1") according to one of the claims 1 to 5, **characterised in that** it is coated in part chemically neutral.

7. Process for emptying of the container (1, 1', 1") according to one of the claims 1 to 6, **characterised in that** the container will be emptied in one step by pressure with a thumb onto the container.

## Revendications

1. Récipient (1, 1', 1") pour les médicaments fluides à appliquer, surtout les médicaments parentéraux, comprenant une enveloppe (6, 6') intégrale tubulaire qui est fermée à l'exception d'une ouverture (7) pour le médicament, et qui est agencé tel que la délivrance du médicament est mis en oeuvre par varier au moins une région de l'enveloppe (6, 6'), lequel récipient comprend en outre une canule (9, 10) au région de l'ouverture (7), laquelle canule (9) et formée dans une manière intégrale avec l'enveloppe tubulaire (6, 6'),
**caractérisé par le fait que** le récipient (1, 1', 1") a un volume inférieure à 5 ml et l'enveloppe (6, 6') du récipient (1, 1', 1") est au moins partiellement formé dans une manière flexible et qu'il a une section relativement flexible et rigide courbée vers l'extérieure et qui va flexiblement sauter vers l'intérieur de l'enveloppe sous pression extérieure après avoir dépassée sa courbure et va exercer une action de déplacement permanente sur le médicament qui est dans le récipient, tel que le récipient peut être vidé à la fois par serrage simplement avec le pouce et tel qu'après l'initiation de la délivrance du médicament par exercer la pression la délivrance va s'effectuer indépendamment.

2. Récipient selon la revendication 1 **caractérisé par le fait qu'**il est fuselé à partir de son bout d'ouverture (7) au deuxième bout soudé en raison de son soudage.

3. Récipient (1, 1', 1") selon l'une des revendications 1 ou 2 **caractérisé par le fait qu'**il est composé de plastique, surtout de polypropylène, PE, tel que le LDPE or PET.

4. Récipient (1, 1', 1 ") selon l'une des revendications 1 à 3, **caractérisé par le fait que** 'il a un stratifié.

5. Récipient (1, 1', 1") selon l'une des revendications 1 à 4, **caractérisé par le fait que** 'il est formé au moins partiellement dans une manière transparente.

6. Récipient (1, 1', 1") selon l'une des revendications 1 à 5, **caractérisé par le fait qu'**il est revêtu partiellement dans une manière chimiquement neutre.

7. Procédé pour vider le récipient (1, 1', 1") selon l'une des revendications 1 à 6, **caractérisé par le fait que** le récipient est vidé à la fois par serrage avec le pouce.
